# EUROPÄISCHE PATENTSCHRIFT

(11) **EP 3 597 246 B1**
(45) Veröffentlichungstag und Bekanntmachung des Hinweises auf die Patenterteilung: **05.01.2022**
(21) Anmeldenummer: 18861000.0
(22) Anmeldetag: 04.06.2018
(51) Int. Cl.: A61M 5/46, A61M 5/00, A61M 5/32, A61M 5/31

(54) **SET ZUR VERBESSERUNG EINER SPRITZE**
SET FOR IMPROVING SYRINGE
COMPLEXE DE PERFECTIONNEMENT DE SERINGUE

(30) Priorität: 27.09.2017 RU 2017133628
(43) Veröffentlichungstag der Anmeldung: 22.01.2020
(73) Patentinhaber: Volkov, Daniil Vasilievitch, Mineralnye Vody 357202 (RU)
(72) Erfinder: Volkov, Daniil Vasilievitch, Mineralnye Vody 357202 (RU)
(74) Vertreter: Jeck, Anton
(86) Internationale Anmeldenummer: PCT/RU2018/000361
(87) Internationale Veröffentlichungsnummer: WO 2019/066682

(56) Entgegenhaltungen:
- WO-A2-2004/103431
- WO-A2-2013/138555
- JP-A- S56 500 914
- US-A- 4 373 526
- US-A- 4 760 847
- US-A1- 2009 259 179
- US-A1- 2009 259 179
- US-A1- 2015 032 062

## Beschreibung

Die Erfindung betrifft das Gebiet der medizinischen Geräte, und zwar einen Spritzen-Upgrade-Bausatz gemäß Anspruch 1. Die Erfindung soll Schmerzen während einer Injektion verringern, indem sie die Qualität der Injektion verbessert und die Durchführung des Injektionsverfahrens erleichtert sowie die Länge der in den Körper eingetauchten Spritzennadel genau reguliert.

Bekannte, der Erfindung am Nächsten kommende Analoga sind in ihrer Struktur komplex. Diese Analoga führen nur eine der beiden Funktionen des Bausatzes aus.

Die Funktion der Erfindung zur Vereinfachung und Verbesserung der Qualität der Injektion wird in ähnlicher Weise bei komplexen vorgefüllten Einwegspritzen gelöst (US 61/740207) oder in komplexen wiederverwendbaren Geräten, in denen sich eine Einwegspritze befindet.

Die Funktion der präzisen Regulierung der Länge der in den Körper eingetauchten Nadel wird von komplexen, einstellbaren, wiederverwendbaren Vorrichtungen ausgeführt (KR 2468830), oder es werden scheibenförmige Stopper verwendet, die an der Spritzennadel angebracht sind. Die Stopper sind nicht einstellbar und stellen eine Gefahr für die Sterilität der Nadel dar, wenn die Möglichkeit der Einstellung erlaubt ist.

US 4373526 A offenbart eine Nadelhülse mit Innenflanschen, die bei der Kontrolle der Injektionstiefe helfen.

WO 2013/138555 A2 offenbart eine Sicherheitsnadelvorrichtung mit einer einziehbaren Hülse, die eine abgestufte Injektionstiefenanzeige besitzt, um das Eindringen der Nadel anzuzeigen.

Die der Erfindung zugrunde liegende Aufgabe besteht darin, den Injektionsvorgang zu erleichtern, die Schmerzen während der Injektion zu verringern und die Länge der in den Körper eingetauchten Nadel verlässlich zu regulieren.

Dieses Problem wird mit erfindungsgemäßen Bausatz-Geräte gelöst.

Das Gerät deckt die Nadel teilweise ab und lässt den Teil der Nadel frei, der in den Körper eingetaucht werden soll. Bei der Durchführung einer Injektion liegt das Gerät auf dem Körper auf. In diesem Fall lässt das Gerät die Nadel nicht vollständig in den Körper einsinken. Aufgrund dessen wird der Effekt einer Erleichterung der Injektion erreicht.

Die erste Vorrichtung ist ein Hohlzylinder mit Flanschen an beiden Enden. An den Enden des Geräts befinden sich Fasen entlang der Innenkante. Der Zylinder enthält in der Mitte einen Teil mit normaler Wandstärke und zwei weitere Teile mit reduzierter Wandstärke. Diese erstrecken sich im mittleren Teil zu den Kanten hin und haben unterschiedliche Längen.

Das zweite Gerät ist eine Standard-Nadelkappe mit Markierungen. Der Beginn der Markierung auf der Nadelkappe fällt mit der Stelle zusammen, an die die Nadelspitze gelangt, wenn die Kappe vollständig von der Nadel getragen ist.

In der Zeichnung ist die Erfindung ein Düsensatz für eine Spritze, der Folgendes umfasst:
1. einen Impulsunterdrücker in zwei Richtungen 1 und
2. eine Standardkappe 7 für die Nadel mit einer Markierung 8 darauf.

Die Erfindung wird durch schematische Zeichnungen veranschaulicht, die den gesamten Umfang der Ansprüche dieser technischen Lösung nicht abdecken und darüber hinaus nicht einschränken, sondern nur veranschaulichende Materialien für einen bestimmten Ausführungsfall darstellen:

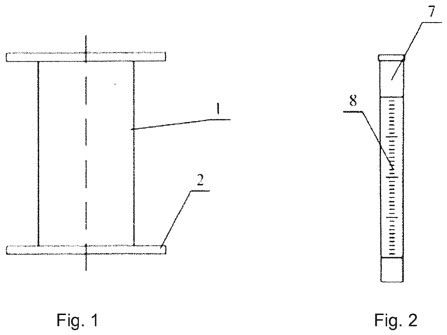

- Fig. 1: ist der Pulsabsorber zweiseitig;
- Fig. 2: ist die Kappe für die Nadel mit der Markierung versehen;

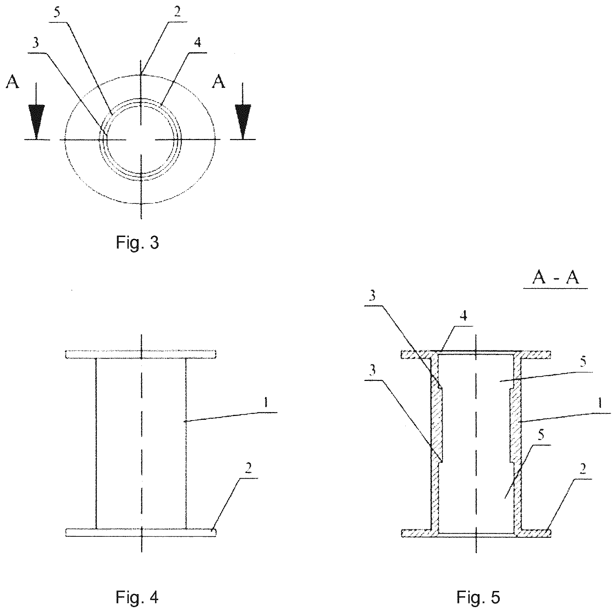

- Fig. 3: ist der Pulsabsorber zweiseitig, Blick von oben;
- Fig. 4: ist der Pulsabsorber zweiseitig, Seitenansicht;
- Fig. 5: ist der Pulsabsorber zweiseitig, Seitenansicht und Längsschnitt,

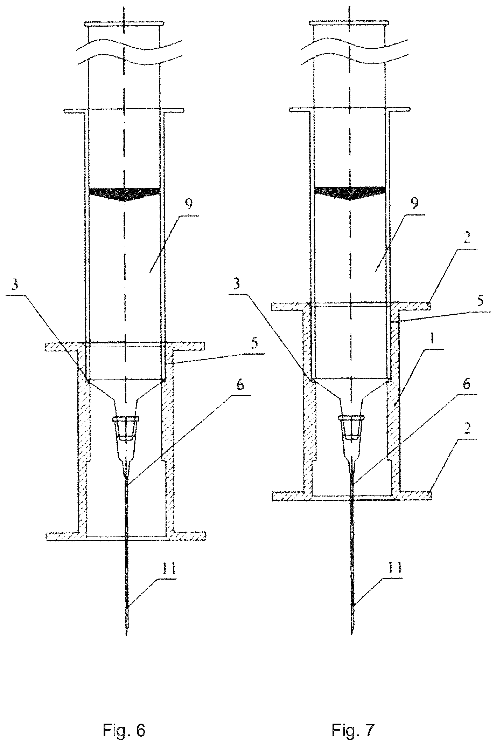

- Fig. 6: ist ein doppelseitiger Pulsabsorber an einer Spritze angebracht, um eine kürzere Nadellänge zu erreichen, Längsschnitt;
- Fig. 7: ist ein Zweiwege-Pulsabsorber auf eine Spritze aufgezogen, um eine längere Nadellänge zu erzielen, Längsschnitt;

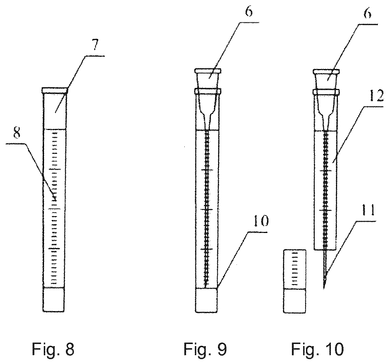

- Fig. 8: ist die Kappe für die Nadel mit einer Markierung versehen;
- Fig. 9: ist eine Kappe für eine Nadel mit einer Markierung mit einer darin eingesetzten Nadel dargestellt;
- Fig. 10: ist eine abgeschnittene Kappe für eine Nadel mit einer Markierung versehen, in die eine Nadel eingesetzt ist

In den Zeichnungen wird die folgende Liste von Elementen verwendet:
1 Hohlzylinder (erste Vorrichtung);
2 Flansche
3 Anschlag
4 Fasen
5 Sitze
6 Nadel
7 Kappe (zweite Vorrichtung)
8 Markierung der Kappe
9 Spritze
10 Markierung starten
11 freies Ende der Nadel
12 Kappe abschneiden

Der Bausatz wird wie folgt verwendet. Die erste Vorrichtung 1 ist ein Hohlzylinder 1 (Rohr) mit Flanschen 2 an beiden Enden. Der Hohlzylinder 1 wird von der Seite der Nadel 6 bis zum Anschlag 3, der sich an der Innenfläche der Vorrichtung befindet, mäßig dicht auf die Spritze 9 aufgesetzt. Die Vorrichtung 1 bleibt in der Arbeitsposition in Bezug auf die Spritze 9 stationär. An den Enden der Vorrichtung 1 entlang der Innenkante befinden sich Abschrägungen 4 zum leichteren Anbringen der Vorrichtung 1 an der Spritze 9. Die Vorrichtung 1 deckt teilweise die Nadel 6 ab, wobei ein Teil der Nadel 11, der in den Körper eingetaucht werden soll, aus der Vorrichtung 1 herausragt. Bei der Durchführung der Injektion liegt die Vorrichtung 1 am Körper an und verhindert, dass die Nadel 6 vollständig in den Körper eintaucht. Aufgrund dessen wird das Einbringen der Nadel 6 in den Körper erleichtert und erfolgt sanft.

An beiden Enden der Vorrichtung 1 befinden sich Flansche 2. Die Flansche 2 dienen zum Löschen und Ableiten der Aufprallkraft der Vorrichtung 1 auf die Oberfläche des Körpers bei scharfem Einführen der Nadel 6 aufgrund der vergrößerten Kontaktfläche der Vorrichtung 1 mit dem Körper. Die Flansche 2 dienen zur besseren Kontrolle der Spritze 9 bei der Verabreichung des Arzneimittels durch Verringerung der axialen Verschiebung, da die Vorrichtung mit ihrer Auflagefläche fest am Körper anliegt. Die Flansche 2 dienen dazu, die Vorrichtung bequem auf die Spritze 9 aufzusetzen. Die Form der Flansche 2 haben vorzugsweise keine regelmäßige runde Form, um das Abrollen der Vorrichtung 1 von glatten Oberflächen zu verhindern, wenn die Vorrichtung 1 getrennt von der Spritze 9 ohne Verpackung liegt.

Da ein Teil der Vorrichtung 1 die Teilungsskala der Spritze 9 teilweise bedeckt, ist bevorzugt, ein transparentes Material für die Herstellung der Vorrichtung 1 auszuwählen.

Die Vorrichtung 1 weist zwei unterschiedlich tiefe Sitze 5 zum Aufsetzen der Spritze 9 auf, einen auf jeder Seite. Bei der Wahl eines der Sitze 5 wird eine unterschiedliche Länge der in den Körper eingetauchten Nadel 11 erreicht. Die Sitze 5 sind in Form einer Verringerung der Dicke der Wände der Vorrichtung 1 von der Innenfläche hergestellt. Somit enthält der Zylinder 1 in der Mitte einen Teil mit normaler Wandstärke, der als Widerlager 3 dient. Der Zylinder 1 enthält zwei Teile mit verringerter Wandstärke an den Rändern, die unterschiedliche Längen haben und als Sitze 5 dienen.

Der Innendurchmesser des Sitzes 5 stimmt mit dem Außendurchmesser des bei der Vorrichtung der Spritze 9 verwendeten Arbeitszylinders überein.

Mit dieser Vorrichtung 1 können zwei weitere bevorzugte Optionen für die Länge der in den Körper eingetauchten Nadel 11 für die damit verwendete Spritze ausgewählt werden.

Die zweite Vorrichtung 7 dient der genaueren Auswahl der Eintauchlänge der Nadel 6. Dazu wird die Markierung 8 auf die Standardkappe für die Nadel 6 gesetzt. Anhand der Markierung kann der Benutzer die Tiefe des Eintauchens der Nadel 1 bestimmen.

Die Kappe 12 wird zur Nadel 6 zurückgeschnitten, die mit der Spritze 9 verbunden ist, um dadurch das gewünschte Ergebnis zu erzielen. Der Einfachheit halber fällt der Beginn der Markierung 10 mit dem Punkt zusammen, an dem die Spitze der Nadel 6 erreicht ist, wenn die Kappe 7 vollständig auf die Nadel 6 aufgesetzt ist. In diesem Fall wird die genaue Größe der Nadel 11 unter der Kappe 7 gelöst. Das Gerät funktioniert ähnlich wie das erste Gerät. Bei der Durchführung der Injektion liegt die abgeschnittene Kappe 12 am Körper an und verhindert, dass die Nadel 6 über die erforderliche Tiefe eintaucht. Während des Abschneidens der Kappe 7 kann ein Pulsabsorber 1 auf die Spritze 9 aufgesetzt werden, um zu verhindern, dass die Nadel 6 irgendetwas berührt.

Durch die Verwendung einer Reihe von Verbesserungen an der Spritze wird somit eine signifikante Steigerung der Qualität der Injektionen erreicht.

## Patentansprüche

1. Bausatz zur Verbesserung einer Spritze (9), bestehend aus zwei Vorrichtungen (1, 7), wobei die Spritze (9) mit einer der beiden Vorrichtungen (1, 7) oder mit beiden Vorrichtungen (1, 7) ausgestattet werden kann, wobei die erste Vorrichtung ein Hohlzylinder (1) mit Flanschen (2) an beiden Enden ist, wobei sich entlang der Innenkante an den Enden der ersten Vorrichtung (1) jeweils Fasen (4) befinden, wobei der Hohlzylinder (1) in der Mitte einen Teil mit normaler Wandstärke aufweist und sich zwei Teile mit verringerter Wandstärke jeweils von dem mittleren Teil zu den Enden erstrecken, welche unterschiedliche Längen haben und jeweils Sitze (5) zum Aufsetzen der Spritze (9) bilden, wobei die zweite Vorrichtungeine Standardkappe (7) eine Spritzennadel (6) der Spritze (9) ist und Markierungen (8) auf der Kappe (7) angebracht sind und wobei der Beginn (10) der Markierung (8) auf der zweiten Vorrichtung (7) mit der Stelle zusammenfällt, an die eine Spitze (11) der Spritzennadel (6) gelangt, wenn die Kappe vollständig von der Spritzennadel (6) getragen ist.

## Claims

1. Kit for improving a syringe consisting of two devices (1, 7),
wherein the syringe (9) can be equipped with one of the two devices (1, 7), the first device being a hollow cylinder (1) with flanges at both ends, there being chamfers (4) along the inner edge at the ends of the first device (1), respectively, the hollow cylinder (1) having a part with normal wall thickness in the middle and two parts with reduced wall thickness extending from the middle part to the ends, respectively, which have different lengths and respectively form seats (5) for fitting the syringe (9), wherein the second device is a standard cap (7) for a syringe needle (6) of the syringe (9), and markings (8), and wherein the beginning (10) of the marking on the second device (7) coincides with the place where a tip (11) of the nail arrives when the cap is fully supported by the syringe needle (6).

## Revendications

1. Kit pour l'amélioration d'une seringue (9) se composant de deux dispositifs (1,7) où la seringue (9) peut être dotée d'un des deux dispositifs (1,7) ou des deux dispositifs (1,7) où le premier dispositif est un cylindre creux (1) avec des brides (2) aux deux extrémités où des chanfreins (4) se situent respectivement aux extrémités du premier dispositif tout au long du bord intérieur est caractérisé de sorte que le cylindre creux (1) présente au milieu une part avec une épaisseur de paroi normale et respectivement deux parts avec une épaisseur de paroi réduite s'étendant de la part moyenne aux extrémités présentant des longueurs différentes formant respectivement des sièges (5) pour la mise en place de la seringue (9) où le deuxième dispositif (7) est une capsule standard (7) pour une aiguille de seringue (6) de la seringue (9) et des marquages sont installés sur cette dernière (8) et où le début (10) du marquage (8) sur le deuxième dispositif (7) coïncide avec la partie rejoignant la seringue (11) de l'aiguille de seringue (6) si le capuchon est porté à titre complet par l'aiguille de seringue (6).
